# EUROPEAN PATENT APPLICATION

(11) **EP 3 753 495 A1**
(43) Date of publication of application: **23.12.2020**
(21) Application number: 19382512.2
(22) Date of filing: 20.06.2019
(51) Int. Cl.: A61B 17/00, A61B 17/42

(54) **PATCH FOR SEALING AN AMNIOTIC MEMBRANE AND DEVICE FOR PLACING SAID PATCH ON AN AMNIOTIC MEMBRANE**

(71) Applicant: Fundació Cellex, 08010 Barcelona (ES); Hospital Sant Joan de Deu, 08950 Esplugues de Llobregat Barcelona (ES); Institut Químic de Sarrià CETS Fundació Privada, 08017 Barcelona (ES); Hospital Clínic de Barcelona, 08036 Barcelona (ES); Institut D'Investigacions Biomèdiques August Pi I Sunyer - IDIBAPS, 08036 Barcelona (ES); Universitat de Barcelona, 08028 Barcelona (Barcelona) (ES)
(72) Inventor: GRATACOS SOLSONA, Eduard, 08036 BARCELONA (ES); EIXARCH ROCA, Elisenda, 08036 BARCELONA (ES); FEBAS BOSOMBA, German, 08017 BARCELONA (ES); BORRÓS GÓMEZ, Salvador, 08017 BARCELONA (ES); MICHELETTI HELFER, Talita, 08028 BARCELONA (ES)
(74) Representative: Herrero & Asociados, S.L.

(57) **Abstract**

Patch (1) for sealing an amniotic membrane, wherein the patch (1) comprises a support (11) and an adhesive (12) that is activated in presence of amniotic liquid or a wet environment.

Device for placing said patch (1) comprising a cannula (2) for inserting said patch (1) in a rolled-up position, said cannula (2) being provided with a handle (3); and a dipstick (4) provided with a pusher (5), said dipstick (4) being inserted inside the cannula (2) in the use position.

The patch adheres efficiently on the amniotic membrane, adapts to the elastic properties of this membrane, and by attaching only to the amnion it does not interfere with the natural sliding movements of one membrane against each other, together with the device to ensure that the patch is placed in the proper position without damaging the membrane.

## Description

The present invention refers to a patch for sealing an amniotic membrane and to a device for placing said patch on an amniotic membrane.

### Background of the invention

Currently, with the improvement of imaging techniques for fetal diagnosis that are used to monitor pregnancy, the detection of fetal anomalies has increased and, therefore, also the need to carry out surgical interventions with which to access directly access the fetus safely.

For some years now, fetoscopy has been used to perform surgeries that may be the only treatment option for several fetal conditions such as twin-to-twin transfusion syndrome and congenital diaphragmatic hernia.

This technique (fetoscopy), by inserting a trocar of different diameters, allows to introduce the necessary tools to perform the corresponding fetal surgery. Access to the fetus is whenever possible limited to a single-entry point, since the higher the number of trocars, the higher complication rate. However, this reduces the potential of the technique to perform more complex operations requiring two or more entry points.

Despite their clear benefits, minimally invasive fetal procedures such as fetoscopy come along with complications, mainly iatrogenic preterm premature rupture of membranes (PPROM), that may affect up to 30% of cases.

This complication is related with the orifice left in the chorio-amniotic membranes once the trocar is removed after finishing the surgery. Human membranes are composed by the amnion (internal) and chorion (external) membranes, which surround the fetus and maintain amniotic fluid. These two membranes are relatively elastic and glide one over each other, which provides a natural defense mechanism. *Ex vivo* and *in vivo* studies have shown that, once perforated, human membranes cannot heal by themselves. Despite that the natural sliding described above represents a defense mechanism, the permanence of the orifice after fetoscopy still results in subsequent rupture of the chorio-amniotic membranes.

This rupture of the membrane associated with fetoscopy has commonly been called iatrogenic Preterm Premature Rupture of Membranes (iPPROM) and is one of the most common complications after fetal surgery. Reported incidences of iPPROM may vary between 30% and 60%, the risk of rupture depending critically on the number and diameter of the trocars. iPPROM is associated with severe consequences including preterm delivery, oligohydramnios, chorioamnionitis, pulmonary hypoplasia, etc., and even the death of the fetus after birth if this is not viable or extremely premature.

With the aim to avoid iPPROM, in recent years it has been tried to seal chorio-amniotic membranes through various strategies and techniques. Some of these experimental treatments range from orifice occlusion with acellular human amnion and collagen plugs, gelatin sponge, etc., to the injection of blood products such as Amniopatch, fibrin glue, Photopolymerized PEG and catechol functionalized PEG, catechol modified Tetronic, etc.

All the strategies above have as common point that they block the natural sliding movements between the amniotic and the chorionic membrane. Recently, an ultrathin polymers film of Poly L-lactic acid was used to deposit an adhesive surface at the internal (amniotic) part of the membrane orifice. Even so, none of these strategies has given optimal results. The systems tested cannot achieve proper adhesion, they are either too soft or too rigid and do not adapt to the elastic properties of the membranes, so that in many cases they may even damage the membranes and worsen clinical results because they impair the natural defense mechanisms of the membranes.

Recently, it has been described a sealing system based in a device composed of an umbrella-shaped polyester coated nitinol mesh and an applicator. The spontaneously unfolding umbrella is pushed through the port, pulled against the amnion, and glued onto the amnion defect site, the glue being applied throughout the two membranes and the uterine wall. In the same way as previous cases, it may not work as the two membranes do not have the physiological sliding movement one over the other since a glue is injected and obstructs the orifice.

Furthermore, the presence of an internal mesh in the umbrella avoids elastic behavior. That is, it does not allow the patch to react naturally to the stress movements of the amnion.

Accordingly, currently there is no suture or sealing system, that can be applied entirely through a fetoscopic (i.e. endoscopic) approach, that avoids the risks of iPPROM derived from fetal surgery. One of the most important associated dangers and on which work has done for years is to somehow prevent the rupture of the chorio-amniotic membranes after the insertion of a trocar by minimally invasive techniques.

### Summary of the invention

The present invention provide a patch that (1) can be introduced through a fetoscopic system, (2) adheres efficiently on the amniotic membrane, (3) adapts to the elastic properties of this membrane, and (4) by attaching only to the amnion it does not interfere with the natural sliding movements of one membrane against each other, together with (5) a device to ensure that the patch is placed in the proper position without damaging the membrane.

With the patch and the device according to the present invention it is possible to solve said drawbacks, providing other advantages that are described below.

The patch for sealing an amniotic membrane according to the present invention comprises a support and an adhesive that is activated in presence of amniotic liquid or a wet environment.

Advantageously, the support is made from an elastomeric material, and it can comprise an outer layer and an inner layer, e.g. the outer layer being made from silicone and the inner layer being made from an elastomeric thermoplastic material that adapts/matches to the elastic modulus of the amnion. This is the reason why it does not break the membrane because it is not too rigid, or it does not detaches because it is not too floppy with respect to the amnion elastic modulus.

According to a preferred embodiment, the adhesive is based in a formulation with (hydroxypropyl) methylcellulose (HPMC), hydroxyethilcellulose (HEC) and a citric acid as a crosslinker.

The patch for sealing an amniotic membrane according to the present invention also comprises advantageously an attachment thread. This thread allows pushing back the patch so it exerts gentle pressure against the amniotic membrane to achieve adhesion, avoiding excessive pressure that might distort and/or damage the chorio-amniotic membranes, factors that would reduce the effectiveness of adhesion.

If desired, the support can also comprise an elastomeric mesh, and preferably the support also comprises a micropattern, which is contact with said adhesive. For example, said micropattern is formed by concave hemispheres.

According to an embodiment, said adhesive can be formed by several adhesive coatings.

Furthermore, the patch can have a semi-lentil shape, even though the shape of the patch can be any suitable shape, such as planar or curved.

The adhesive used in the patch according to the present invention can include a poly-vinylalcohol (PVA) thin layer on top to protect it from the friction and the environment. This layer dissolves quickly in contact with the amniotic fluid and is biocompatible.

According to a second aspect, the present invention also refers to a device for placing a patch as described previously on an amniotic membrane, comprising:
- a cannula for inserting said patch in a rolled-up position, said cannula being provided with an ergonomic handle; and
- a dipstick provided with a pusher, said dipstick being inserted inside the cannula in the use position.

Advantageously, the dipstick is slidably inserted inside the cannula, and the pusher comprises a hole for placing a fastening thread for fastening said patch.

The patch is inserted folded by the device and deployed once inside the uterus. This behavior can be achieved using an elastomer as the main material of the support of the patch. E.g. silicone elastomers are materials approved for use in humans (medical grade silicone).

On the other hand, the amniotic membrane presents mechanical properties clearly different from that of silicone. This is the reason why the union between the patch and the membrane (adhesive) must be able to compensate for mechanical properties between the membrane and the patch.

Furthermore, the patch shape affects the level of adhesion. That is why a semi-lentil shape works better than a totally flat patch.

### Brief description of the drawings

For a better understanding the above explanation and for the sole purpose of providing an example, some non-limiting drawings are included that schematically depict a practical embodiment.
FIG. 1 is a plan view of the patch according to the present invention;
FIG. 2 is a cross-section view of the patch according to the present invention; and
FIG. 3-5 are views showing the use of the device according to the present invention for inserting the patch inside the cannula.

### Description of a preferred embodiment

According to a first aspect, the present invention refers to a patch 1 formed by a biocompatible elastomeric support 11 and a wet-activation adhesive 12 which is activated in presence of amniotic liquid or a wet environment. The elastomeric support 11 and the adhesive 12 will be introduced into the fetal cavity through a trocar once surgery on the fetus has been completed. The patch will remain adhered to the fetal side of the amniotic membrane until the moment of childbirth, without causing any toxicological reaction, either against the mother or against the fetus.

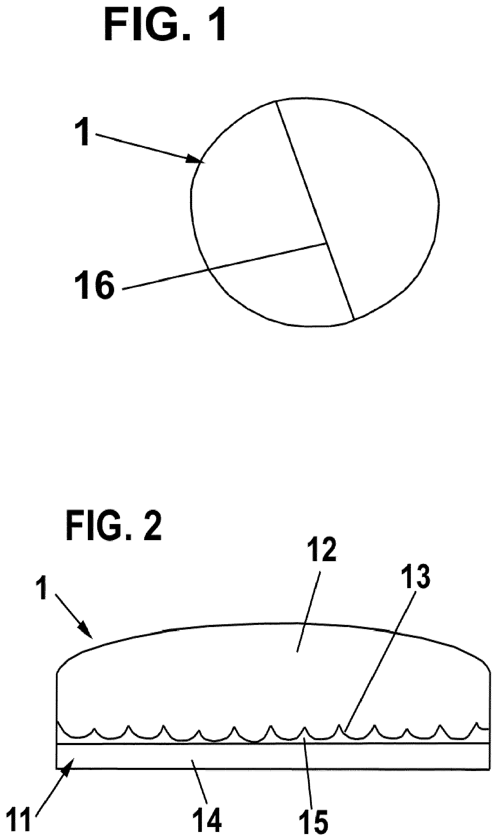

As shown in Fig. 2, the patch 1 is formed by the elastomeric support 11 and the adhesive 12 that is deposited on said support 11, said patch 1 having a flat, curved or semi-lentil shape.

Said support comprises preferably a micropattern 13 with concave hemispheres, e.g. with a deep of 100 µm on one of their sides. In particular, in the case of a semi-lentil patch 1, the micropattern 13 can be located on the outer ring of the inner side, i.e. the side where the adhesive 12 is deposited.

According to a first example, the support 11 is made from a bi-component medical grade silicone (NuSil MED-4950P) which can be colored with dyes for medical use.

According to a second example, the support 11 comprises an outer layer 14 of medical grade silicone and an inner layer 15 of an electrospinned thermoplastic polycarbonate and poly-urethane copolymer, both perfectly joined.

Both examples have an attachment thread 16 (Fig. 1) on its internal part that acts as an attachment point, which is introduced before injecting the silicone. A mesh (not shown in the drawing) with elastomeric properties that acts as an internal skeleton can be added.

The adhesive 12 used in the patch 1 according to the present invention can be an adhesive based in hydroxypropyl methylcellulose (HPMC) or hypromellose (H7509, Sigma-Aldrich). Pharm grade Hypromelose (USP, EP, JP) Metolose® SR, ShinEtsu.

The adhesive 12 used in the patch 1 according to the present invention can be an adhesive based in Hydroxyetylcellullose (HEC) crosslinked with citric acid. Pharm grade Hydroxyethilcellulose (USP, EP, JP) Natrosol™ 250, Ashland™.

The adhesive 12 used in the patch 1 according to the present invention can be an adhesive based in other cellulose derivates and other formulations of the commented cellulose derivatives.

The adhesive 12 used in the patch 1 according to the present invention can be adhesive derivates from naphta or derivates from cyanoacrilates.

The patch 1 according to the present invention can also include bioadhesive coatings. These bioadhesives are composed, by one side, for various thin films of polymers and copolymers made with Chemical Vapor Deposition (CVD) techniques, and for the other side by the activations of dopaminated Hyaluronic Acid (dHA).

Said polymeric or copolymeric coatings have different thickness of reactive molecules as glycidyl methacrylate (GMA), pentafluorophenil methacrilate (PFM), allylisotiocyanate (AITC), and crosslink molecules as hydroxyethilmethacrilate (HEMA), allylamine and diethilenglicol diacrialte (EGDA).

The adhesive 12 can also be based on dopaminated hyaluronic acid (dHA). In this case, the dHA can be modified to increase the percent of chain dopamination. The adhesive properties start when the catechol groups are oxided and become to quinone. Quinones are reactive to make covalent bonds between the other chains and proteins of the amniotic membrane.

Different concentrations and formulations of dHA can be used, e.g. a concentration of 10mg/ml of synthetized dHA and 6 mg/ml of FeCl₃ as an oxidant, both dissolved in sterilized mQ water.

The adhesive 12 used in the patch 1 according to the present invention can include a poly-vinylalcohol (PVA) thin layer on top to protect it from the friction and the environment. This layer dissolves quickly in contact with the amniotic fluid and is biocompatible.

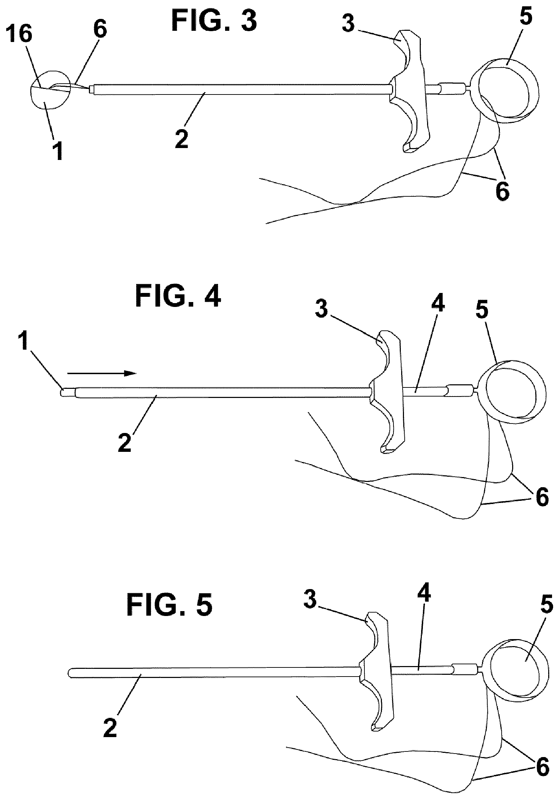

The present invention, according to a second aspect, refers to a device for placing said patch 1, shown in Figs. 3-5. The device comprises a cannula 2 with an ergonomic handle 3 and a dipstick 4 with a finger pusher 5.

According to two alternative embodiments, the ergonomic handle 3 can have a design as a syringe, or a design as a micropipette (shown in Figs. 3-5).

Furthermore, the finger pusher 5 can also have different designs, allowing to push the dipstick 4 with the thumb. The first design allows to push the dipstick 4, while the second design (shown in Figs. 3-5) allows to push and retract the dipstick 4.

In the use position, the dipstick 4 is placed inside the cannula 2, as will be described hereinafter.

The method for placing the patch 1 according to the present invention using the disclosed device is the following:
A first step is inserting the dipstick 4 with the finger pusher 5 into the cannula 2 with the ergonomic handle 3.

Once the patch 1 is ready with the adhesive 12, a fastening thread 6 is passed through the attached thread 16 of the patch 1, returning in the same direction, forming a double fastening thread, as shown in Fig. 3.

The two ends of the fastening thread 6 is inserted through the dipstick 4 and go out through a hole at the finger pusher 5.

Then, the dipstick 4 is partially removed, and the patch 1 is rolled up (Fig. 4) and inserted into the cannula 2 (Fig. 5), while preserving the tension of the double fastening thread 6.

Once inserted into the cannula 2, the patch 1 is placed in the desired position on the amniotic membrane, by the following steps:
1. Introduction of a trocar to realize the fetal surgery, in a conventional way;
2. Extraction of the device with the patch from an esterized blister;
3. Introduction of the device into the trocar after the surgical process is finished, until the final position. This final position allows the cannula 2 to protrude through the terminal end of the trocar.
4. The finger pusher 5 is pushed until the dipstick 4 pushes out the patch 1, and the patch 1 unfolds.
5. Once the adhesive 12 of the patch 1 is rehydrated by the amniotic liquid, the patch 1 is put in contact with the amniotic membrane by a tension of the fastening thread 6 during some time. Then the trocar is removed along with the device, keeping the fastening thread 6 tensioned, so that when the device is removed the thread 6 slides inside the hollow dipstick 4.

It must be pointed out that during the placement of the patch 1 on the amniotic membrane the device is placed in the positions shown in Figs. 3-5, but in the inverse order.

Even though reference has been made to a specific embodiment of the invention, it is obvious for a person skilled in the art that the patch and the device described herein are susceptible to numerous variations and modifications, and that all of the details mentioned can be substituted for other technically equivalent ones without departing from the scope of protection defined by the attached claims.

## Claims

1. Patch (1) for sealing an amniotic membrane, **characterized in that** the patch (1) comprises a support (11) and an adhesive (12) that is activated in presence of amniotic liquid or a wet environment.

2. Patch (1) for sealing an amniotic membrane according to claim 1, wherein the patch (1) also comprises an attachment thread (16).

3. Patch (1) for sealing an amniotic membrane according to anyone of the previous claims, wherein the support (11) is made from an elastomeric material.

4. Patch (1) for sealing an amniotic membrane according to anyone of the previous claims, wherein the support (11)comprises an outer layer (14) and an inner layer (15).

5. Patch (1)for sealing an amniotic membrane according to claim 4, wherein the outer layer (14) is made from silicone and the inner layer (15) is made from a thermoplastic material.

6. Patch (1) for sealing an amniotic membrane according to anyone of the previous claims, wherein the adhesive (12) is based in a formulation of (hydroxypropyl) methylcellulose (HPMC) and hydroxyethylcellulose (HEC).

7. Patch (1) for sealing an amniotic membrane according to anyone of the previous claims, wherein the support (11) also comprises a mesh.

8. Patch (1) for sealing an amniotic membrane according to anyone of the previous claims, wherein the support (11) also comprises a micropattern (13), which is contact with said adhesive (12).

9. Patch (1) for sealing an amniotic membrane according to claim 8, wherein said micropattern (13) is formed by concave hemispheres.

10. Patch (1) for sealing an amniotic membrane according to anyone of the previous claims, wherein said adhesive (12) is formed by several adhesive coatings.

11. Patch (1) for sealing an amniotic membrane according to anyone of the previous claims, wherein the patch (1) has a semi-lentil shape.

12. Patch (1) for sealing an amniotic membrane according to anyone of the previous claims, wherein the adhesive (12) includes a poly-vinylalcohol (PVA) layer.

13. Device for placing a patch (1) according to anyone of the previous claims on an amniotic membrane, **characterized in that** comprises:
- a cannula (2) for inserting said patch (1) in a rolled-up position, said cannula (2) being provided with a handle (3); and
- a dipstick (4) provided with a pusher (5), said dipstick (4) being inserted inside the cannula (2) in the use position.

14. Device according to claim 13, wherein the dipstick (4) is slidably inserted inside the cannula (2).

15. Device according to claim 13 or 14, wherein the pusher (5) comprises a hole for placing a fastening thread (6) for fastening said patch (1).
